# EUROPÄISCHE PATENTSCHRIFT

(11) **EP 1 035 851 B1**
(45) Veröffentlichungstag und Bekanntmachung des Hinweises auf die Patenterteilung: **16.08.2001**
(21) Anmeldenummer: 98965685.5
(22) Anmeldetag: 21.11.1998
(51) Int. Cl.: A61K 31/495

(54) **ENDOTHELIN-REZEPTORANTAGONISTEN ZUR BEKÄMPFUNG VON HYPERLIPIDÄMIE**
ENDOTHELIN RECEPTOR ANTAGONISTS FOR COMBATING HYPERLIPIDAEMIA
ANTAGONISTES DU RECEPTEUR A L'ENDOTHELINE POUR LUTTER CONTRE L'HYPERLIPIDEMIE

(30) Priorität: 05.12.1997 DE 19754082
(43) Veröffentlichungstag der Anmeldung: 20.09.2000
(73) Patentinhaber: KNOLL AKTIENGESELLSCHAFT, 67061 Ludwigshafen (DE)
(72) Erfinder: MÜNTER, Klaus, D-68163 Mannheim (DE); KIRCHENGAST, Michael, D-68161 Mannheim (DE)
(74) Vertreter: Dörper, Thomas Michael, Dr.
(86) Internationale Anmeldenummer: EP9807501
(87) Internationale Veröffentlichungsnummer: WO9929308

(56) Entgegenhaltungen:
- EP-A- 0 670 320
- WO-A-97/08169
- ALLCOCK, GRAHAM H. ET AL: "Role of endothelin receptors in the regional and systemic vascular responses to ET-1 in the anaesthetized ganglion-blocked rat: Use of selective antagonists." BRITISH JOURNAL OF PHARMACOLOGY, (1995) VOL. 116, NO. 5, PP. 2482-2486. , XP002110589
- KADDOURA, SAMER (1) ET AL: "Endothelin -1 is involved in norepinephrine-induced ventricular hypertrophy in vivo: Acute of bosentan, an orally active, mixed endothelin ET-A and ET-B receptor antagonist." CIRCULATION, (1996) VOL. 93, NO. 11, PP. 2068-2079. , XP002110590
- BIRD, J. EILEEN (1) ET AL: "A role for endothelin ET-A receptors in regulation of renal function in spontaneously hypertensive rats." EUROPEAN JOURNAL OF PHARMACOLOGY, (1995) VOL. 294, NO. 1, PP. 183-189. , XP002110591
- DATABASE WPI Section Ch, Week 9840 Derwent Publications Ltd., London, GB; Class B03, AN 98-462777 XP002111591 & JP 10 194972 A (TANABE SEIYAKU CO), 28. Juli 1998 (1998-07-28)
- PARRINELLO, GASPARE (1) ET AL: "Central obesity and hypertension: The role of plasma endothelin." AMERICAN JOURNAL OF HYPERTENSION, (1996) VOL. 9, NO. 12 PART 1, PP. 1186-1191. , XP002110592
- GALLUZZI, F. (1) ET AL: "Evaluation of endothelin -1 plasmatic levels in obese children: Relationship with insulinemia, fibrinogen and lipid profile." HORMONE RESEARCH (BASEL), (1997) VOL. 48, NO. SUPPL. 2, PP. 183. MEETING INFO.: 5TH JOINT MEETING OF THE EUROPEAN SOCIETY FOR PAEDIATRIC ENDOCRINOLOGY AND THE LAWSON WILKINS SOCIETY FOR PEDIATRIC ENDOCRINOLOGY, IN COLLABORATION WITH THE AUSTRALIAN PA, XP002110593
- HAUNER, H.: "Prevention of adipose tissue growth." INTERNATIONAL JOURNAL OF OBESITY, (1994) VOL. 18, NO. SUPPL. 2, PP. 147. MEETING INFO.: 7TH INTERNATIONAL CONGRESS ON OBESITY TORONTO, ONTARIO, CANADA AUGUST 20-25, 1994 , XP002110594

## Beschreibung

Die vorliegende Erfindung betrifft die Bekämpfung von Hyperlipidämie bzw. von Krankheiten, die durch Hyperlipidämie hervorgerufen werden.

Das Peptidhormon Endothelin ist wegen seiner starken vasokonstriktorischen Eigenschaften bekannt. Endothelin-Rezeptorantagonisten werden deshalb überwiegend in cardiovaskulären Krankheitsbildern getestet.

Gegenstand der Erfindung ist die Verwendung von Endothelin-Rezeptorantagonisten zur Herstellung von Arzneimitteln zur Bekämpfung von Hyperlipidämie bzw. von Krankheiten, die durch Hyperlipidämie hervorgerufen werden.

Als Endothelin-Rezeptorantagonisten können sowohl EndothelinA-als auch gemischte Endothelin_{A/B}-Rezeptorantagonisten verwendet werden.

Als Endothelin-Rezeptorantagonisten kommen insbesondere
1. TBC-11251 (J. Med. Chem., 40, No. 11, 1690-97, 1997),
2. BMS-193884 (EP 558.258)
3. BMS-207940 (Pharmaprojects (13.06.97)),
4. BQ-123 (Exp. Opin. Invest. Drugs, 1997, 6, No. 5, 475-487),
5. SB-209670 (Exp. Opin. Invest. Drugs, 1997, 6, No. 5, 475-487),
6. SB-217242 (Exp. Opin. Invest. Drugs, 1997, 6, No. 5, 475-487),
7. SB-209598 (Trends in Pharmacol. Sci., 17, 177-81, 1996),
8. TAK-044 (Exp. Opin. Invest. Drugs, 1997, 6, No. 5, 475-487),
9. Bosentan (Trends in Pharmacol. Sci., 18, 408-12, 1997),
10.PD-156707 (J. Med. Chem., 40, No. 7, 1063-74, 1997),
11.L-749329 (Bioorg. Med. Chem. Lett., 7, No. 3, 275-280, 1997),
12.L-754142 (Exp. Opin. Invest. Drugs, 1997, 6, No. 5, 475-487),
13.ABT-627 (J. Med. Chem., 40, No. 20, 3217-27, 1997),
14.A-127772 (J. Med. Chem., 39, No. 5, 1039-1048, 1996),
15.A-206377 (213^{th} American Chemical Society National Meeting, San Francisco, California, USA, 13 - 17 April, 1997, Poster, MEDI 193),
16.A-182086 (J. Med. Chem., 40, No. 20, 3217-27, 1997),
17.EMD-93246 (211^{th} American Chemical Society National Meeting, New Orleans, USA, 1996, Poster, MEDI 143),
18.EMD-122801 (Bioorg. Med. Chem. Lett., 8, No. 1, 17-22, 1998),
19.ZD-1611 (Trends in Pharmacol. Sci., 18, 408-12, 1997),
20.AC-610612 (R&D Focus Drug News (18.05.98)),
21.T-0201 (70th Annual Meeting of the Japanese Pharmacological Society, Chiba, Japan, 22-25 March 1997, Lecture, O-133),
22.J-104132 (R&D Focus Drug News (15.12.97)) und insbesondere 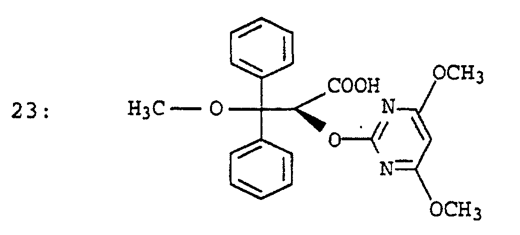 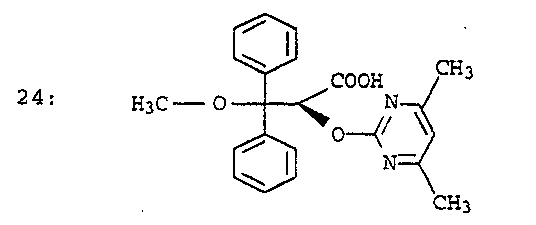 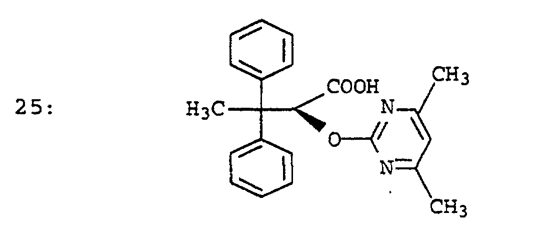 und 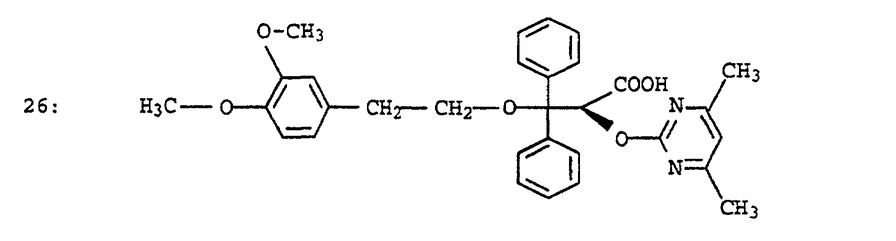 in Frage.

Man spricht von Fettleibigkeit, wenn das Körpergewicht mindestens 20 % über dem Normalgewicht liegt. Die Ursachen der Fettleibigkeit sind Übernährung oder falsche Nahrungsverwertung, z.B. familiäre Hypercholesterolämie. Als Krankheiten, die durch Fettleibigkeit hervorgerufen werden bzw. damit häufig einhergehen, sind speziell Hypertonie, Typ 2 Diabetes, Hyperlipidämie, chronisches Nierenversagen und Arteriosklerose und möglicherweise auch Gicht zu nennen.

In Tierversuchen ließ sich das Krankheitsbild der Fettleibigkeit bisher nur sehr schwierig nachstellen (Verabreichung extrem hoher Cholesterindosen). In letzter Zeit wurden jedoch knock-out Mäuse gezüchtet, denen das Gen für Apolipoprotein E fehlt und die zur Testung von Substanzen gegen Fettleibigkeit eingesetzt werden können.

In dem Tiermodell der apoE-knockout Maus wurde die Wirkung des Endothelinrezeptorantagonisten Substanz 23 untersucht. In Kontrollratten stieg wie zu erwarten das Körpergewicht der Tiere unter einer fettreichen Ernährung stark an. Verbunden damit war ein Größenwachstum der Leber mit gleichzeitiger Verfettung der Leber. In einer Parallelgrupe wurden die Tiere mit der Substanz 23 (50 mg/kg/d) behandelt. In dieser Gruppe wurde die Zunahme des Körper- und Lebergewichts vollkommen verhindert. Auch histologisch war die Leber unauffällig.

Die Endothelin_{A}- und Endothelin_{A/B}-Rezeptorantagonisten müssen lebenslang appliziert werden. Ihre Dosierung beträgt 50 bis 500 mg pro Patient und Tag.

Die Endothelin_{A}- und Endothelin_{A/B}-Rezeptorantagonisten werden im allgemeinen oral, z.B. in Form von Tabletten, Lacktabletten, Dragees, Hart- und Weichgelatinekapseln, Lösungen, Emulsionen oder Suspensionen verabreicht. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragees und Hartgelatinekapseln kann eine erfindungsgemäße Kombination mit pharmazeutisch inerten, anorganischen oder organischen Excipientien verarbeitet werden. Als solche Excipientien kann man für Tabletten, Dragees und Hartgelatinekapseln Lactose, Maisstärke oder Derivate davon, Talk, Stearinsäure oder deren Salze verwenden. Für Weichgelatinekapseln eignen sich als Excipientien pflanzliche Öle, Wachse, Fette, halbfeste und flüssige Polyale.

Zur Herstellung von Lösungen und Sirupen eignen sich als Excipientien z.B. Wasser, Polyole, Saccharose, Invertzucker, Glukose und dergleichen. Für Injektionslösungen eignen sich als Excipientien Wasser, Alkohole, Polyole, Glyzerin, vegetabile Öle. Für Suppositorien eignen sich als Excipientien natürliche oder gehärtete Öle, Wachse, Fette, halbflüssige oder flüssige Polyole und dergleichen.

Die pharmazeutischen Zubereitungen können daneben noch Konservierungsmittel, Lösevermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel und/oder Antioxidantien enthalten.

Die nachfolgenden Beispiele erläutern die Erfindung.

### Beispiel 1

Es wurden Lacktabletten folgender Zusammensetzung hergestellt:

| | |
|---|---|
| Verbindung 23 | 300,0 mg |
| Lactose wasserfrei | 30,0 mg |
| Mikrokristalline Cellulose | 30,0 mg |
| Polyvinylpyrrolidon | 20,0 mg |
| Magnesiumstearat | 5,0 mg |
| Polyethylenglykol 6000 | 0,8 mg |
| Eisenoxid gelb | 1,2 mg |
| Titandioxid | 0,3 mg |
| Talk | 0,7 mg |

Verbindung 23, die Lactose, die Cellulose und das Polyvinylpyrrolidon werden feuchtgranuliert und getrocknet. Das gesiebte Granulat wird mit dem Magnesiumstearat gemischt, und die preßfertige Mischung zu ovalen Tablettenkernen zu je 390,0 mg verpreßt. Anschließend werden die Kerne mit Hilfe eines Lackierverfahrens überzogen, bis die Lacktabletten ein Endgewicht von 400 mg erreicht haben.

### Beispiel 2

Analog Beispiel 1 wurden Lacktabletten hergestellt, die jedoch statt 300 mg Verbindung 23 300 mg Verbindung 26 enthielten.

### Beispiel 3

Herstellung von Hartgelatinekapseln folgender Zusammensetzung:

| | |
|---|---|
| Verbindung 23 | 250,0 mg |
| Lactose krist. | 18,0 mg |
| Polyvinylpyrrolidon | 15,0 mg |
| Mikrokristalline Cellulose | 17,5 mg |
| Natrium-carboxymethylstärke | 10,0 mg |
| Talk | 0,7 mg |
| Magnesiumstearat | 3,0 mg |

Die ersten fünf Bestandteile werden feuchtgranuliert und getrocknet. Das Granulat wird mit der Natrium-carboxymethylstärke, dem Talk und dem Magnesiumstearat gemischt und die Mischung in Hartgelatinekapseln der Größe 0 abgefüllt.

## Patentansprüche

1. Vezwendung von Endothelin-Rezeptorantagonisten zur Herstellung von Arzneimitteln zur Bekämpfung von Hyperlipidämie.

## Claims

1. The use of endothelin receptor antagonists for producing drugs for controlling hyperlipidemia.

## Revendications

1. Utilisation d'antagonistes des récepteurs de l'endothéline pour la préparation de médicaments prévus pour le traitement de l'hyperlipidémie.
